**Europäisches Patentamt**

**European Patent Office**

**Office européen des brevets**

(19)

(11) Veröffentlichungsnummer: **0 000 054**
**B1**

(12) **EUROPÄISCHE PATENTSCHRIFT**

(51) Int. Cl.³: **C 07 C 1/36,**
**C 07 C 11/00,**
**C 07 C 13/00,**
**C 07 C 13/20,**
**C 07 C 13/12,**
**C 07 C 11/10**

(21) Anmeldenummer: **78100096.3**

(22) Anmeldetag: **06.06.78**

(54) **Verfahren zur Herstellung von olefinisch ungesättigten aliphatischen oder cycloaliphatischen Kohlenwasserstoffen**

(30) Priorität: **10.06.77 DE 2726106**

(43) Veröffentlichungstag der Anmeldung:
**20.12.78 Patentblatt 78/01**

(45) Bekanntmachung des Hinweises auf die Patenterteilung:
**29.10.80 Patentblatt 80/22**

(84) Benannte Vertragsstaaten:
**BE DE FR GB NL**

(56) Entgegenhaltungen:
**FR - A - 1 274 507**
**US - A - 2 034 872**
**US - A - 2 310 762**
**US - A - 2 383 205**
**Chemical Abstracts, Volume 84, (1976) 16774a**
**Chemical Abstracts, Volume 84, (1976) 58587x.**

(73) Patentinhaber: **BASF Aktiengesellschaft**
**Carl-Bosch-Strasse 38**
**D – 6700 Ludwigshafen (DE)**

(72) Erfinder: **Horn, Peter, Dr.**
**Im Bruennel 20**
**D - 6945 Hirschberg (DE)**
**Grosskinsky, Otto-Alfred, Dr.**
**Semmelweisstrasse 8**
**D - 6700 Ludwigshafen (DE)**
**Fuchs, Hugo, Dr.**
**Egellstrasse 28**
**D - 6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

### Verfahren zur Herstellung von olefinisch ungesättigten aliphatischen oder cycloaliphatischen Kohlenwasserstoffen

Gegenstand der Erfindung ist ein Verfahren zur Herstellung von olefinisch ungesättigten aliphatischen oder cycloaliphatischen Kohlenwasserstoffen durch Erhitzen von Alkancarbonsäuren mit 3 bis 20 Kohlenstoffatomen, Alkandicarbonsäuren mit 4 bis 20 Kohlenstoffatomen oder 5- oder 6-gliedrigen Cycloalkancarbonsäuren oder der jeweiligen Alkyl-, Cycloalkyl-, Aralkyl- oder Phenylester auf Temperaturen von 250 bis 800°C in der Gasphase in Gegenwart von Katalysatoren.

Häufig ist es erwünscht aus Carbonsäuren, die beispielsweise bei Oxidationsverfahren erhalten werden oder aus Carbonsäuren oder deren Estern, die bei Carbonylierungsverfahren als Nebenprodukte anfallen, die entsprechenden Olefine durch Decarbonylierung zu gewinnen. Die Decarbonylierung von Carbonsäuren oder deren Ester ist dann von besonderem Interesse, wenn schwerzugängliche Olefine aus leichter zugänglichen Carbonsäuren oder deren Estern hergestellt werden können.

Aus der DT-AS 1 158 050 ist zwar schon bekannt, daß man Carbonsäuren durch Erhitzen auf 250 bis 800°C, in der Gasphase in Gegenwart von Katalysatoren aus Oxiden, Hydroxiden und Phosphaten der 2. bis 4. Hauptgruppe und der 3. bis 8. Nebengruppe des Periodensystems in Säureanhydride und/oder Ketone überführen kann. Diese Arbeitsweise gestattet es jedoch nicht, zu den entsprechenden Olefinen zu gelangen.

Es war deshalb die technische Aufgabe gestellt, ein Verfahren zur Verfügung zu stellen, bei dem man aus Carbonsäuren oder deren Estern durch Decarbonylierung die entsprechenden Olefine zu erhalten.

Diese Aufgabe wird gelöst in einem Verfahren zur Herstellung von olefinisch ungesättigten aliphatischen oder cycloaliphatischen Kohlenwasserstoffen durch Erhitzen von Alkancarbonsäuren mit 3 bis 20 Kohlenstoffatomen, Alkandicarbonsäuren mit 4 bis 20 Kohlenstoffatomen oder 5- bis 6-gliedrigen Cycloalkancarbonsäuren oder der jeweiligen Alkyl-, Cycloalkyl-, Aralkyl- oder Phenylester auf Temperaturen von 250 bis 800°C in der Gasphase in Gegenwart von Katalysatoren, wobei man Katalysatoren verwendet deren katalytisch aktive Masse aus Bortrioxid, Borsäure und/oder Bornitrid sowie mindenstens aus einem der Oxide von Aluminium, Silicium, Zinn, Blei, Titan oder Zirkonium besteht.

Das neue Verfahren hat den Vorteil, daß auf einfache Weise Carbonsäuren oder deren Ester in Olefine übergeführt werden können. Ferner hat das neue Verfahren den Vorteil, daß es leicht in einen technischen Maßstab übertragbar ist und mit guten Ausbeuten und Umsätzen verläuft.

Bevorzugt geht man von Alkancarbonsäuren mit 3 bis 16 Kohlenstoffatomen, Alkandicarbonsäuren mit 4 bis 12 Kohlenstoffatomen der Cyclohexancarbonsäure aus. Geeignet sind auch deren Alkyl-, Cycloalkyl-, Aralkyl- oder Phenylester. Insbesondere deren $C_1$- bis $C_8$-Alkylester, Cyclohexylester, Benzylester oder Phenylester. Besonders bevorzugt werden die freien Carbonsäuren oder die entsprechenden Methylester als Ausgangsstoffe verwendet. Besondere technische Bedeutung hat das neue Verfahren für die Herstellung von Cyclohexen aus Hexahydrobenzoesäure oder deren Estern. Geeignete Ausgangsstoffe sind beispielsweise Propionsäure, Propionsäuremethylester, Isobuttersäure, oder Isobuttersäurebutylester, Capronsäure, Capronsäuremethylester, Dekancarbonsäure, Palmitinsäure, Palmitinsäuremethylester, Adipinsäuredimethylester, Methylglutarsäuredimethylester, · Cyclopentancarbonsäuremethylester, Hexahydrobenzoesäure, Hexahydrobenzoesäuremethylester, Hexahydrobenzoesäurecyclohexylester, Hexahydrobenzoesäurephenylester oder Hexahydrobenzoesäurebenzylester.

Es versteht sich, daß man bei der Decarbonylierung von Monocarbonsäuren oder deren Ester zu einfach olefinisch ungesättigte Verbindungen mit einem Kohlenstoffatom weniger gelangt, während man bei der Decarbonylierung von Dicarbonsäuren zu Diolefinen mit 2 Kohlenstoffatomen weniger gelangt.

Die Umsetzung wird in der Gasphase durchgeführt. Zweckmäßig verdampft man die als Ausgangsstoffe verwendeten Carbonsäuren oder deren Ester. Gegebenenfalls verwendet man, um die Ausgangsstoffe besser in der Gasphase anwenden zu können, zusätzlich Inertgase wie Stickstoff, Edelgase, Kohlendioxid, Kohlenmonoxid, Wasserdampf oder Rauchgase. Die Menge der mitverwendeten Inertgase ist nicht kritisch, da sie lediglich als Trägergase dienen.

Vorteilhaft führt man die Umsetzung bei Atmosphärendruck, geringem Überdruck oder vermindertem Druck z.B. bis zu 26,5 mbar durch. Besonders bewährt hat sich ein Druckbereich von Atmosphärendruck bis 133 mbar.

Die Umsetzung verläuft bei Temperaturen von 250 bis 800°C. Vorteilhaft wendet man Temperaturen von 250 bis 700°C an.

Die Umsetzung wird in Gegenwart von Katalysatoren durchgeführt, deren katalytisch aktive Masse aus Bortrioxid, Borsäure und/oder Bornitrid sowie mindestens einem der Oxide von Aluminium, Silizium, Blei, Titan oder Zirkonium besteht. Bevorzugte katalytische Massen enthalten 10 bis 60, insbesondere 20 bis 55 Gewichtsprozent an Bortrioxid, Borsäure und-oder Bornitrid. Der Rest der katalytisch aktiven Masse besteht dann aus einem der genannten Oxide. Besonders bewährt haben sich katalytische Massen, die Bortrioxid oder Borsäure insbesondere Bortrioxid enthalten.

Zu den bevorzugten Oxiden, die außerdem in der katalytisch aktiven Massen enthalten sind, gehören Aluminiumoxid wie Hydrargillit, Böhmit oder Bayerit sowie deren Entwässerungsprodukte, z.B. $\gamma$, $\varepsilon$, oder $\delta$-Aluminiumoxid, ferner Zinndioxid, Titandioxid oder Kieselsäuregel. Besonders bevorzugt werden Aluminiumoxid und Titandioxid, in der Anatasmodifikation, verwendet. Besondere technische Bedeutung hat $\alpha$-Aluminiumoxid erlangt, da es die Isomerisierung der gebildeten olefinisch ungesättigten Kohlenwasserstoffe vermindert.

Erfindungsgemäße Katalysatoren sind auch solche, die durch Phasenbildung einer der verwendeten Borverbindungen mit den genannten Oxiden entstanden sind, z.B. aus Bortrioxid und Aluminiumoxid wie Verbindungen der Summenformel $9\ Al_2O_3 . 2\ B_2O_3$ oder $2\ Al_2O_3 . B_2O_3$. Röntgenographisch läßt sich in solchen Phasen Aluminiumoxid nicht mehr nachweisen.

Vorteilhaft enthalten die katalytisch aktiven Massen zusätzlich mindestens eines der Elemente der VIII. Gruppe des Periodensystems Mangan, Chrom, Kupfer, Zink, Cadmium, Silber und/oder Gold. Vorzugsweise sind die genannten Elemente in den katalytisch aktiven Massen enthalten in Mengen von 0,1 bis 10 Gewichtsprozent, bezogen auf die katalytisch aktive Masse, berechnet als Metall.

Geeignete Katalysatoren werden beispielsweise hergestellt, indem man Aluminiumoxid sowie Bortrioxid oder Borsäure gegebenenfalls unter Zusatz von Verbindungen der oben genannten Schwermetalle, wie Mangannitrat, Manganacetat, Kobaltnitrat, Kobaltacetat, Nickelnitrat, Rhodiumchlorid, Tris(triphenylphosphin)chlorrhodium, Iridiumchlorid, Palladiumnitrat, Platinchlorid, Zinknitrat, Cadmiumnitrat, Kupfernitrat, Silbernitrat oder Goldchlorid mischt, gegebenenfalls mit Wasser anteigt und verknetet und zu Formlingen wie Pillen, Tabletten oder Strängen verformt. Vorteilhaft wird die Masse anschließend getrocknet und zweckmäßig bei Temperaturen bis 400°C getempert. Falls die katalytisch aktive Masse als Wirbelschichtkatalysator verwendet werden soll, ist es empfehlenswert, die Formlinge auf die gewünschte Teilchengröße, z.B. auf eine Durchschnittskorngröße von 0,01 bis 2 mm, insbesondere 0,2 bis 1 mm, zu brechen. Anschließend wird die Katalysatormasse auf Temperaturen von 600 bis 1500, vorzugsweise 700 bis 1300, insbesondere 1000 bis 1200°C über einen Zeitraum von 15 Minuten bis 20 Stunden, insbesondere 30 Minuten bis 5 Stunden erhitzt.

Nach einer anderen vorteilhaften Arbeitsweise gelangt man zu den erfindungsgemäßen katalytisch aktiven Massen, indem man beispielsweise Aluminiumoxid oder eines der anderen genannten Oxide, die bereits eine Körnung von 0,1 bis 2 mm haben, unter Druck mit Bortrioxid oder Borverbindungen, die unter den Herstellungsbedingungen des Katalysators in Bortrioxid oder Borsäure übergehen, unter Mitverwendung von Solvatisierungsmitteln bei erhöhter Temperatur unter Druck tränkt. Als Solvatisierungsmittel eignet sich vorteilhaft Wasser, gegebenenfalls unter Zusatz geringer Mengen an Ammoniak oder Mineralsäuren wie Chlorwasserstoffsäure oder Perchlorsäure, ferner sind geeignete Solvatisierungsmittel Alkohole wie Methanol, Äthanol oder Glycerin. Vorteilhaft tränkt man die Oxide unter erhöhtem Druck, z.B. 1,1 bis 20 bar bei erhöhter Temperatur, z.B. 50 bis 250°C. Das Gewichtsverhältnis von Lösungsmittel zu den verwendeten Ausgangsstoffen beträgt vorteilhaft 1:1 bis 10:1. Insbesondere verwendet man gleiche Gewichtsteile Lösungsmittel und Ausgangsstoffe. Einen solchen sogenannten Tränkkatalysator trocknet man anschließend bei Temperaturen von 50 bis 200°C und erhitzt anschließend auf Temperaturen von 600 bis 1500°C. Die Katalysatoren können als Vollkatalysatoren oder auf Träger aufgebracht angewandt werden.

Die Katalysatoren können fest angeordnet verwendet werden. Vorteilhaft wendet man die Katalysatoren jedoch in ab- und aufwerbelnder Bewegung in einem Wirbelbett an. Es hat sich als vorteilhaft erwiesen, wenn man bei der Umsetzung Verweilzeiten von 0,01 bis 50 Sekunden am Katalysator einhält.

Das Verfahren wird beispielsweise ausgeführt, indem man einen feinkörnigen Trägerkatalysator, beispielsweise Aluminiumoxid, das Bortrioxid enthält, in einer zur Erzeugung eines Wirbelbetts geeigneten Vorrichtung wirbelt, auf die angegebenen Temperaturen erhitzt und von unten Carbonsäureester oder Carbonsäuren in gasförmiger Form, gegebenenfalls zusammen mit Trägergasen durch das Katalysatorbett leitet. Selbstverständlich kann die Carbonsäure oder der Ester auch flüssig in das erhitzte Katalysatorbett eingeleitet werden.

Das erhaltene Gasgemisch wird abgekühlt und hierbei Olefine nichtumgesetzte Ausgangsstoffe und gegebenenfalls bei der Verwendung von Estern die entsprechenden Alkohole kondensiert. Die inerten gasförmigen Anteile können wieder als Trägergas verwendet werden. Desgleichen können nichtumgesetzte Ausgangsstoffe wieder der Reaktion zugeführt werden. Gegebenenfalls ist es notwendig, die anfallenden Olefine von den als Nebenprodukten erzeugten Alkoholen abzutrennen, z.B. durch Destillation.

Olefinisch ungesättigte Kohlenwasserstoffe, die nach dem Verfahren der Erfindung hergestellt werden, eignen sich beispielsweise zur Herstellung von Aldehyden nach der Oxosynthese. Cyclohexen, das man nach dem Verfahren der Erfindung erhält, eignet sich zur Herstellung von Cyclohexanol, wie beispielsweise in der GB—PS 339 592 beschrieben wird.

Das Verfahren nach der Erfindung sei in folgenden Beispielen veranschaulicht.

## Beispiel 1

Aus einem Verdampfer wurden 200 g Hexahydrobenzoesäure je Stunde in einen auf 550°C beheizten Wirbelschichtreaktor eingeleitet, der mit 630 g Katalysator, bestehend aus 52,3 Gewichtsprozent $\gamma$—$Al_2O_3$ und 45,5 Gewichtsprozent $B_2O_3$ mit einer Korngröße von 0,1 bis 0,3 mm und einem Schüttgewicht von 0,63 kg je Liter beschickt war. Die Wirbelung des Katalysators wurde durch den Hexahydrobenzoesäuredampf, einen schwachen Stickstoffstrom, sowie durch Aufrechterhaltung eines Unterdruckes von 480 mbar gewährleistet. Die entstandenen Reaktionsdämpfe wurden kondensiert. Nach 3-stündigem Betrieb wurden folgende Produkte isoliert: 86 g Wasser, 384 g einer organischen Phase. Die organische Phase wurde mit wäßriger Natriumbicarbonat-Lösung gewaschen, um Spuren nicht umgesetzter Hexahydrobenzoesäure zu entfernen, und anschließend destilliert. Die gaschromatographische Untersuchung des Destillats ergab folgende Zusammensetzung: 63 Gewichtsprozent Cyclohexen, 29 Gewichtsprozent 1-Methylcyclopenten (1), 4 Gewichtsprozent 3-Methylcyclopenten (1) und 4 Gewichtsprozent 4-Methylcyclopenten (1).

## Beispiel 2

Es wurde analog Beispiel 1 verfahren, mit dem Unterschied, daß der im Beispiel 1 verwendete Katalysator vor Verwendung 4 Stunden auf 1150°C erhitzt wurde. Nach 7-stündigem Betrieb wurden 61 g Wasser sowie 356 g einer organischen Phase, bestehend nach Aufarbeitung gemäß Beispiel 1 aus 72 Gewichtsprozent Cyclohexen und 23 Gewichtsprozent 1-Methyl-cyclopenten (1), erhalten.

## Beispiel 3

Es wurde analog Beispiel 1 verfahren, mit dem Unterschied, daß der in Beispiel 1 verwendete Katalysator zusätzlich 1,3 Gewichtsprozent Rhodium enthält. Nach 3-stündigem Betrieb wurden 69 g Wasser und 366 g einer organischen Phase, bestehend nach Aufarbeitung gemäß Beispiel 1 aus 74 Gewichtsprozent Cyclohexen, 19,6 Gewichtsprozent 1-Methylcyclopenten (1) und 5,5 Gewichtsprozent Benzol, erhalten.

## Beispiel 4

Aus einem Verdampfer wurden 300 g Hexahydrobenzoesäure je Stunde in einen auf Reaktionstemperatur (480°C) beheizten Wirbelschichtreaktor eingeleitet, der mit 795 g Katalysator, bestehend aus 35 Gewichtsprozent $\gamma$—$Al_2O_3$, 45 Gewichtsprozent $B_2O_3$ und 20 Gewichtsprozent $SnO_2$ mit einer Korngröße von 0,1 bis 0,3 mm und einem Schüttgewicht von 0,795 kg je Liter beschickt war. Die Wirbelung des Katalysators wurde durch den Hexahydrobenzosäuredampf, einen schwachen Stickstoffstrom, sowie durch Aufrechterhaltung eines Unterdruckes von 480 mbar gewährleistet. Die entstehenden Reaktionsdämpfe wurden kondensiert. Nach 4-stündigem Betrieb wurden 186 g Wasser und 813 g einer organischen Phase, bestehend nach Aufarbeitung gemäß Beispiel 1 aus 68,3 Gewichtsprozent Cyclohexen und 19,2 Gewichtsprozent 1-Methylcyclopenten (1) isoliert.

## Beispiel 5

Aus einem Verdampfer wurden 200 g Hexahydrobenzoesäuremethylester je Stunde in einen auf Reaktionstemperatur (550°C) beheizten Wirbelschichtreaktor eingeleitet, der mit 630 g Katalysator, bestehend aus 52,3 Gewichtsprozent $\gamma Al_2O_3$ und, 45,5. Gewichtsprozent $B_2O_3$ mit einer Korngröße von 0,1 bis 0,3 mm und einem Schüttgewicht von 0,63 kg je Liter beschickt war. Die Wirbelung des Kontaktes wurde durch den Hexahydrobenzoesäuremethylesterdampf, einen schwachen Stickstoffstrom, sowie durch Aufrechterhaltung eines Unterdruckes von 480 mbar gewährleistet. Die entstandenen Reaktionsdämpfe wurden kondensiert. Nach 3-stündigem Betrieb der Anlage wurden 184 g einer organischen Phase, bestehend nach Wasserwäsche und Destillation aus 82,3 Gewichtsprozent Cyclohexen und 17,7 Gewichtsprozent 1-Methylcyclopenten (1), isoliert.

## Beispiel 6

Es wurde analog Beispiel 3 verfahren, außer daß die Reaktionstemperatur 700°C betrug. Nach 3-stündigem Betrieb der Anlage wurden 265 g einer organischen Phase, bestehend nach Wasserwäsche und Destillation aus 74 Gewichtsprozent Cyclohexen und 25 Gewichtsproztent 1-Methylcyclopenten (1), isoliert.

## Beispiel 7

Aus einem Verdampfer wurden 200 g Hexahydrobenzoesäurecyclohexylester je Stunde in einen auf Reaktionstemperatur (600°C) beheizten Wirbelschichtreaktor eingeleitet, der mit 630 g Katalysator, bestehend aus 52,3 Gewichtsprozent $\gamma$—$Al_2O_3$ und 45,5 Gewichtsprozent $B_2O_3$ mit einer Korngröße von 0,1 bis 0,3 mm und einem Schüttgewicht von 0,63 kg je Liter, beschickt war. Die Wirbelung des Kontaktes wurde durch den Hexahydrobenzoesäurecyclohexylesterdampf, einen schwachen Stickstoffstrom, sowie durch Aufrechterhaltung eines Unterdruckes von 480 mbar gewährleistet. Die entstandenen Reaktionsdämpfe wurden kondensiert. Nach 3-stündigem Betrieb der Anlage wurden 440 g einer organischen Phase erhalten, welche nach Wasserwäsche und Destillation aus 54 Gewichtsprozent 1-Methylcyclopenten (1) und 42,6 Gewichtsprozent Cyclohexen bestand.

## Beispiel 8

Es wurde analog Beispiel 4 verfahren, außer daß als Ester Hexahydrobenzoesäurephenylester eingesetzt wurde und die Reaktionstemperatur 700°C betrug. Nach 3-stündigem Betrieb der Anlage wurden 243 g Phenol, und 266 g einer organischen Phase, bestehend nach Wasserwäsche und Destillation aus 62 Gewichtsprozent 1-Methylcyclopenten (1) und 32 Gewichtsprozent Cyclohexen, isoliert.

### Beispiel 9

Es wurde analog Beispiel 4 verfahren, außer daß als Ester Hexahydrobenzoesäurebenzylester eingesetzt wurde und die Reaktionstemperatur 700°C betrug. Nach 3-stündigem Betrieb wurden 77 g Toluol, 31 g Benzaldehyd, 51 g Benzylalkohol, und 122 g einer organischen Phase, bestehend nach Wasserwäsche und Destillation aus 44 Gewichtsprozent 1-Methylcyclopenten (1) und 36 Gewichtsprozent Cyclohexen, isoliert.

### Beispiel 10

Aus einem Verdampfer wurden 200 g Capronsäure je Stunde in einen auf Reaktionstemperatur (550°C) beheizten Wirbelschichtreaktor eingeleitet, der mit 630 g Katalysator bestehend aus 50 Gewichtsprozent $B_2O_3$ und 50 Gewichtsprozent $\gamma$—$Al_2O_3$ mit einer Korngröße von 0,1 bis 0,3 mm und einem Schüttgewicht von 0,63 kg pro Liter beschickt war. Die Wirbelung des Katalysators wurde durch den Capronsäuredampf, einen schwachen Stickstoffstrom sowie durch Aufrechterhaltung eines Unterdruckes von 480 mbar gewährleistet. Die entstandenen Reaktionsdämpfe wurden kondensiert. Nach 3-stündigem Betrieb der Anlage wurden 229 g einer organischen Phase, bestehend aus 1,5 Gewichtsprozent 3-Methylbuten (1), 14,3 Gewichtsprozent Penten (1), 10 Gewichtsprozent 2-Methyl-buten (1), 30 Gewichtsprozent Penten (2) trans, 17,2 Gewichtsprozent Penten (2) cis, 21 Gewichtsprozent 2-Methyl-buten (2), isoliert.

### Patentansprüche

1. Verfahren zur Herstellung von olefinisch ungesättigten aliphatischen oder cycloaliphatischen Kohlenwasserstoffen durch Erhitzen von Alkancarbonsäuren mit 3 bis 20 Kohlenstoffatomen, Alkandicarbonsäuren mit 4 bis 20 Kohlenstoffatomen oder 5- oder 6-gliedrigen Cycloalkancarbonsäuren oder der jeweiligen Alkyl-, Cycloalkyl-, Aralkyl- oder Phenylester auf Temperaturen von 250 bis 800°C in der Gasphase in Gegenwart von Katalysatoren, *dadurch gekennzeichnet*, daß man Katalysatoren verwendet, deren katalytisch aktive Masse aus Bortrioxid, Borsäure und/oder Bornitrid sowie mindestens aus einem der Oxide vor Aluminium, Silizium, Zinn, Blei, Titan oder Zirkonium besteht.

2. Verfahren nach Anspruch 1, *dadurch gekennzeichnet*, daß man Temperaturen von 250 bis 700°C einhält.

3. Verfahren nach den Ansprüchen 1 und 2, *dadurch gekennzeichnet*, daß die Katalysatoren einen Gehalt von 10 bis 60 Gewichtsprozent Bortrioxid, Borsäure und/oder Bornitrid, bezogen auf die katalytisch aktive Masse, enthalten.

4. Verfahren nach den Ansprüchen 1 bis 3, *dadurch gekennzeichnet* daß man Aluminiumoxid verwendet.

5. Verfahren nach den Ansprüchen 1 bis 4, *dadurch gekennzeichnet*, daß man $\alpha$-aktive Masse zusätzlich einen Gehalt an mindestens einem der Elemente der VIII. Gruppe des Periodensystems, Mangan, Chrom, Kupfer, Zink, Cadmium, Silber oder Gold hat.

6. Verfahren nach den Ansprüchen 1 bis 4, *dadurch gekennzeichnet*, daß man Aluminiumoxid verwendet.

7. Verfahren nach den Ansprüchen 1 bis 4, *dadurch gekennzeichnet*, daß man $\alpha$-Aluminiumoxid verwendet.

8. Verfahren nach den Ansprüchen 1 bis 7, *dadurch gekennzeichnet*, daß man Hexahydrobenzoesäure oder deren Alkyl-, Cycloalkyl-, Aralkyl- oder Phenylester als Ausgangsstoffe verwendet.

9. Verfahren nach den Ansprüchen 1 bis 7, *dadurch gekennzeichnet*, daß die Mischphasen der Katalysatoren der Zusammensetzung 9 $Al_2O_3$ . 2 $B_2O_3$ oder 2 $Al_2O_3$ . $B_2O_3$ entsprechen.

### Claims

1. A process for the manufacture of olefinically unsaturated aliphatic or cycloaliphatic hydrocarbons by heating alkanecarboxylic acids of 3 to 20 carbon atoms, alkanedicarboxylic acids of 4 to 20 carbon atoms or 5- or 6-membered cycloalkanecarboxylic acids or the respective alkyl, cycloalkyl, aralkyl or phenyl esters at 250 to 800°C in the gas phase in the presence of catalysts, *characterized in that* catalysts are used whose catalytically active material consists of boron trioxide, boric acid and/or boron nitride and at least one of the oxides of aluminium, silicon, tin, lead, titanium and zirconium.

2. A process as claimed in claim 1, *characterized in that* temperatures of 250 to 700°C are used.

3. A process as claimed in claims 1 and 2, *characterized in that* the catalysts contain 10 to 60 percent by weight of boron trioxide, boric acid and/or boron nitride, based on the catalytically active material.

4. A process as claimed in claims 1 to 3, *characterized in that* boron trioxide is used.

5. A process as claimed in claims 1 to 4, *characterized in that* the catalytically active material additionally contains at least one of the elements of Group VIII of the Periodic System, manganese, chromium, copper, zinc, cadmium, silver and gold.

6. A process as claimed in claims 1 to 4, *characterized in that* aluminium oxide is used.

7. A process as claimed in claims 1 to 4, *characterized in that* $\alpha$-aluminium oxide is used.

8. A process as claimed in claims 1 to 7, *characterized in that* hexahydrobenzoic acid or alkyl, cycloalkyl, aralkyl or phenyl esters thereof are used as starting materials.

9. A process as claimed in claims 1 to 7, *characterized in that* the mixed phases of the catalysts have the compositions $9 Al_2O_3 . 2 B_2O_3$ or $2 Al_2O_3 . B_2O_3$.

**Revendications**

1. Procédé de préparation d'hydrocarbures à insaturation oléfinique aliphatiques ou cycloaliphatiques, par chauffage d'acides alcanecarboxyliques comportant 3 à 20 atomes de carbone, d'acides alcanedicarboxyliques comportant 4 à 20 atomes de carbone, ou d'acides cycloalcanecarboxyliques à 5 ou 6 membres, ou leurs alkyl-cycloalkyl-aralkyl- ou phénylesters, à des températures de 250 à 800°, en phase gazeuse, en présence de catalyseurs, caractérisé par le fait que l'on utilise des catalyseurs dont la masse active catalytique est constituée de trioxyde de bore, d'acide borique et de nitrure de bore, ou seulement de nitrure de bore, ainsi qu'au moins un des oxydes d'aluminium, silicium, étain, plomb, titane ou zirconium.

2. Procédé selon la revendication 1, dans lequel on maintient des températures de 250 à 700°C.

3. Procédé selon l'une des revendications 1 et 2, dans lequel les catalyseurs ont une teneur de 10 à 60% en poids de trioxyde de bore, d'acide borique et de nitrure de bore ou seulement de nitrure de bore, rapportée à la masse catalytique active.

4. Procédé selon l'une des revendications 1 à 3, dans lequel on utilise du trioxyde de bore.

5. Procédé selon l'une des revendications 1 à 4, dans lequel la masse active catalytique contient en plus au moins un des éléments du VIIIème groupe tu tableau périodique, manganèse, chrome, cuivre, zinc, cadmium, argent ou or.

6. Procédé selon l'une des revendications 1 à 4, dans lequel on utilise de l'oxyde d'aluminium.

7. Procédé selon l'une des revendications 1 à 4, dans lequel on utilise de l'oxyde d'aluminium-$\alpha$.

8. Procédé selon l'une des revendications 1 à 7, dans lequel on utilise des acides hexahydrobenzoïques ou leurs alkyl-, cycloalkyl-, aralkyl- ou phénylesters comme produit de départ.

9. Procédé selon l'une des revendications 1 à 7, dans lequel la phase mélange des catalyseurs correspond à la composition $9 Al_2O_3 . 2 B_2O_3$ ou $2 Al_2O_3 . B_2O_3$.